# EUROPEAN PATENT SPECIFICATION

(11) **EP 4 079 317 B1**
(45) Date of publication and mention of the grant of the patent: **15.10.2025**
(21) Application number: 20901632.8
(22) Date of filing: 17.12.2020
(51) Int. Cl.: A61K 38/06, A61K 8/64, A61P 7/02, A61P 17/00, A61P 43/00, A61Q 19/00

(54) **GLUTATHIONE-CONTAINING COMPOSITION**
GLUTATHION-HALTIGE ZUSAMMENSETZUNG
COMPOSITION CONTENANT DU GLUTATHION

(30) Priority: 18.12.2019 JP 2019228188
(43) Date of publication of application: 26.10.2022
(73) Proprietor: Shiseido Company, Ltd., Chuo-ku Tokyo 104-0061 (JP)
(72) Inventor: NAKANISHI Shinobu, Tokyo 104-0061 (JP); DENDA Mitsuhiro, Tokyo 104-0061 (JP)
(74) Representative: Ter Meer Steinmeister & Partner
(86) International application number: PCT/JP2020/047265
(87) International publication number: WO 2021/125291

(56) References cited:
- WO-A1-2019/131406
- WO-A1-99/16411
- NAKANISHI SHINOBU ET AL: "Glutathione Counteracts the Effects of Japanese Cedar (<i>Cryptomeria japonica</i>) Pollen Allergen Cry j1", vol. 43, no. 10, 1 October 2020 (2020-10-01), JP, pages 1591 - 1594, XP093084109, ISSN: 0918-6158, Retrieved from the Internet <URL:https://www.jstage.jst.go.jp/article/bpb/43/10/43_b20-00264/_pdf/-char/en> DOI: 10.1248/bpb.b20-00264
- KUHNAU J. ET AL.: "Glutathion, Schwermetalle und Blutgerinnung", HOPPE-SEYLER'S Z. PHYSIOL. CHEM., vol. 227, no. 1 - 4, 01-1934, pages 145 - 168

## Description

### Technical Field

The present invention relates to a technical field for improving skin condition, specifically to cosmetics, cosmetology or medical technology.

### Background Art

Since the skin is located in the outermost layer of the living body, it is constantly exposed to stimuli from the outside world. Stimuli from the outside world include physical stimuli such as ultraviolet rays and temperature, as well as stimuli b0036y chemical substances and antigenic substances. Cedar pollen, which causes pollinosis, acts through the mucous membrane to cause respiratory symptoms and pharyngeal symptoms that are specific to pollinosis, and also causes cedar pollen dermatitis through contact with the skin. Cryj1 which is known as a sugi antigen has been reported to cause a decrease in lamellar granules in keratinocytes (Non-patent Document 1). The lamellar granules are granules found in the cytoplasm of keratinocytes in the stratum granulosum, and are secreted extracellularly by causing apoptosis of keratinocytes. The lamellar granules contain intercellular lipids such as ceramide, cholesterol and fatty acids; and a decrease in lamellar granules results causes a reduction in the skin barrier function.

On the other hand, the present inventors have reported in a previous study that tranexamic acid blocks the delay in thrombin-mediated recovery of the epidermal permeation barrier, which is induced by the cedar pollen allergen Cryj1 (Non-patent Document 2, Patent Document 1).

### Citation List

### Patent Documents

Patent Document 1: International Publication Pamphlet WO 2019/131406

### Non-patent Documents

Non-patent Document 1: Arch Dermatol Res 308:49-54, 2016
Non-patent Document 2: Scientific Reports (2018)8, 15610

### Summary of the Invention

### Problems to be Solved by the Invention

An objective of the present invention is to provide a novel thrombin action inhibitor, a composition for preventing pollen-induced skin roughness, and a cosmetic or non-therapeutic method for preventing pollen-induced skin roughness.

### Means for Solving the Problems

The present inventors conducted diligent studies to identify substances that inhibit signal transduction which suppresses the secretion of lamellar granules starting from the activation of PAR-1 by thrombin or thrombin-like protein in response to pollen antigen, and they discovered for the first time that glutathione acts as an effective inhibitor, that is, glutathione or a salt thereof can be used as an active ingredient of a thrombin action inhibitor. The inventors invented a composition for preventing pollen-induced skin roughness, which comprises glutathione or a salt thereof, and a cosmetic or non-therapeutic method for preventing pollen-induced skin roughness.

Therefore, the present invention is defined by the appended claims.

### Effects of the Invention

Glutathione or a salt thereof can be used to inhibit the thrombin action. Furthermore, glutathione or a salt thereof can be used to prevent skin roughness caused by pollen.

### Brief Description of the Drawings

[FIG. 1] Figure 1 is a graph showing the suppression of thrombin activity by glutathione. The test was performed using glutathione at concentrations of 500 µg/ml, 1 mg/ml and 2.5 mg/ml for a 40 ng/ml solution of prothrombin.
[FIG. 2] Figure 2 shows the quantitative results of calcium imaging over time in cultured keratinocytes, and it is shown that the addition of Cryj1 suppresses the excitation of fluorescence, while the addition of Cryj1 and glutathione (Glutathione) suppresses the excitation.
[FIG. 3] Figure 3 is a graph showing the results of quantifying the intracellular calcium concentration value about 2 minutes after the addition of Cryj1 in the same experimental system as in Figure 2. It has been shown that the addition of glutathione (Glutathione) suppresses the increase of the intracellular calcium concentration (on the right).

### Mode for Carrying Out the Invention

In one aspect of the present invention, the present invention relates to a composition for preventing skin roughness caused by pollen, which comprises glutathione or a salt thereof.

It has been reported that Cryj1, which is an antigen of Japanese cedar pollen, causes a decrease in lamellar granules. The reduction of lamellar granules leads to skin roughness. Previous studies by the present inventors have found that activation of PAR-1 by thrombin is the starting point for signal transduction that suppresses the secretion of lamellar granules.

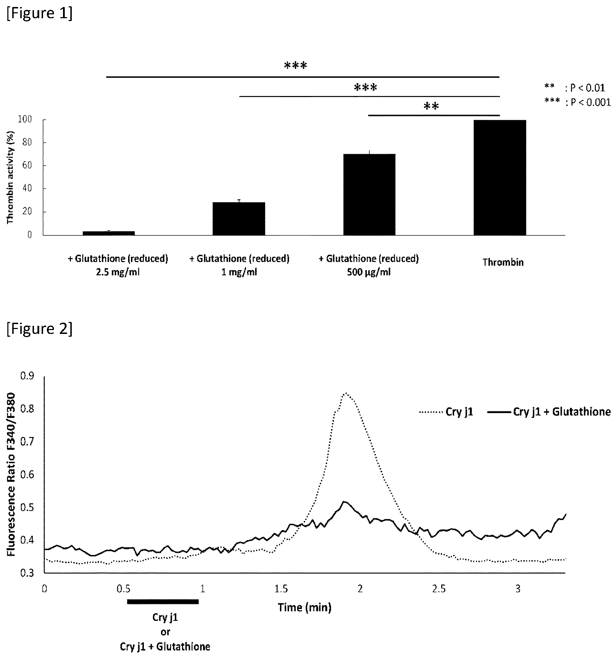

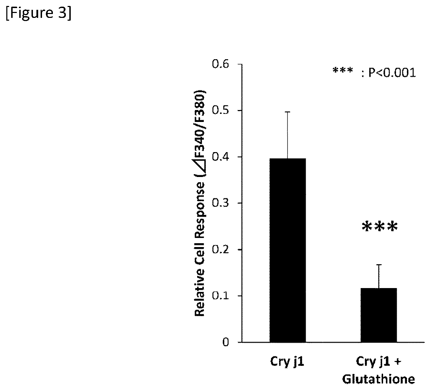

As disclosed in the present specification, the present inventors have found that glutathione can be used to inhibit thrombin action (Figure 1). Then, it was found that the cellular action of Cryj1 on keratinocytes was suppressed by the administration of glutathione (Figures 2 and 3). Therefore, glutathione can be used to prevent, suppress, or reduce the skin roughness caused by pollen.

Glutathione is one of the antioxidants and is known to have an auxiliary role in protecting cells from reactive oxygen species such as free radicals and peroxides. On the other hand, the effect on thrombin action or the effect on skin roughness caused by pollen is not known at all.

In one aspect of the present invention, reduced glutathione or oxidized glutathione is preferably used. Glutathione may also be used in the form of salts. Examples of the salt include acid addition salts, metal salts, ammonium salts, organic amine addition salts, amino acid addition salts and the like. Without limitations thereto, acid addition salts include hydrochlorides, sulfates, citrates, malates, α-ketoglutarates, and the like; metal salts include sodium salts, potassium salts, and the like; amino acid addition salts include aspartic acid, glutamate, and the like.

In one aspect of the present invention, the pollen that causes skin roughness includes, for example, pollen of sugi (Cryptomeria japonica). It is known that various pollens can cause allergic reactions and pollen dermatitis. For example, in Japan, some sort of pollen is scattered throughout the year, such as spring cedar pollen, summer gramineous pollen, and autumn ragweed pollen, but the amount of cedar pollen scattered is particularly large and easily produces an effect on the skin. Therefore, one aspect of the present invention relates to a composition for preventing skin roughness caused by Japanese cedar pollen, which comprises glutathione or a salt thereof. However, as described above, the pollen that can cause skin roughness includes Japanese cypress, Dactylis glomerata, Lolium hybridum, Miscanthus sinensis, Humulus japonicus, Ambrosia artemisiaefolia, Artemisia indica, Alnus japonica and the like in addition to sugi, but the pollen in the context of the present invention is not limited to Japanese cedar pollen.

The mechanism of onset of skin roughness caused by pollen is different from that of skin roughness caused by other reasons. Briefly, in skin roughness caused by other reasons, serine proteases in the epidermis are activated, and the serine proteases further activate the PAR-2 receptor, causing Ca influx into cells, resulting in skin roughness (see, for example, J Invest Dermatol 128, 1930-1939, 2008 and J Invest Dermatol 126, 2074-2086, 2006). In skin roughness caused by pollen, pollen activates thrombin and then activates the PAR-1 receptor, causing Ca influx into cells (see, for example, Scientific Reports (2018) 8, 15610). That is, the skin roughness caused by pollen is clearly distinguished from other skin roughness, and the prevention of skin roughness caused by pollen is recognized as a problem different from the prevention of regular skin roughness.

In another aspect of the present invention, the present invention relates to a thrombin action inhibitor, which comprises glutathione or a salt thereof.

Thrombin is a type of serine protease involved in blood coagulation and is also called factor Ila. In the coagulation system, it catalyzes the reaction of fibrinogen to fibrin and activates factors V, VIII and IX. It is present in blood as prothrombin (factor II) and is activated by the action of factor Xa and factor Va to become thrombin.

In the present specification, inhibition of the thrombin action may result in suppression of the action of thrombin or a thrombin-like protein, including, for example, inhibition of thrombin on the active center as well as inhibition of the prothrombin to thrombin activation. Furthermore, in the present invention, it is particularly preferable that the action of thrombin on keratinocytes is suppressed, and the action on keratinocytes is mainly exerted through activation of the thrombin receptor PAR-1. Therefore, the effect of thrombin inhibition may be an inhibitory effect on the PAR-1 activation. A thrombin-like protein is a protein that activates PAR-1 like thrombin. The thrombin-like protein may be a homologue or ortholog of thrombin, or a protein of non-human origin. Further, the thrombin-like protein may be a partial peptide having thrombin activity of human or non-human prothrombin.

Examples of the thrombin substrate used for measuring the action of thrombin include peptides or proteins capable of degrading thrombin. Such peptides or proteins are labeled with a fluorescent substance and a quencher, and are labeled with a fluorescent substrate or a coloring substance that enables detection of fluorescence only when cleaved by thrombin. Color-developing substrates that enable the detection of absorbance only in the case of cleavage by thrombin are commercially available, and such fluorescent substrates and color-developing substrates can be used.

Thrombin activity can also be measured by measuring the activity of PAR-1-expressing cells. As an example, when PAR-1 is activated, the cell concentration of Ca²⁺ in the cells increases. Therefore, thrombin activity can be measured by performing Ca²⁺ imaging. More specifically, when PAR-1-expressing cells are cultured in a medium containing thrombin and a candidate drug agent and Ca²⁺ imaging is performed, the candidate drug agent can be selected as a substance that suppresses the thrombin action when the increase in the intracellular Ca²⁺ concentration due to thrombin is suppressed. Here, thrombin may be added to the medium, or it may be produced by activating its precursor substance, prothrombin. Prothrombin may be added, or it may be expressed and secreted by the cells themselves. Activation of prothrombin may be carried out by the addition of any antigen, such as Cryj1.

Thrombin action inhibitors, as described in the present specification, can be used to prevent pollen-induced skin roughness. In addition, since it suppresses the reaction caused by allergens, it can be used as an anti-allergic agent, an anti-itch agent, or an anti-inflammatory agent. Skin conditions that can be improved or treated by thrombin action inhibitors include cosmetic problems such as dry skin, sensitive skin, scales, and dandruff. Further, the thrombin action inhibitor can be used for treatment, prevention, improvement and the like of skin diseases such as pollen dermatitis, atopic dermatitis, psoriasis and eczema, as well as allergic diseases such as pollinosis.

The thrombin action inhibitor can be used by any arbitrary administration route including oral administration and intravenous injection, but transdermal administration is preferable from the viewpoint of directly acting on the skin. By transdermal administration, even a drug agent that causes side effects when administered systemically can be tolerated from the viewpoint of improvement of the skin condition, especially prevention of skin roughness.

The thrombin action inhibitor according to the present invention can be blended into functional foods, cosmetics, quasi-drugs, and pharmaceuticals for the purpose of preventing skin roughness caused by pollen. In other words, the composition containing glutathione or a salt thereof according to the present invention can be prepared as a functional food, a cosmetic, a quasi-drug, or a pharmaceutical product. Examples of cosmetics to be blended include sunscreens, skin toner, skin serum, skin cream, aftercare lotions, sun oils, and the like, but any arbitrary cosmetics can be blended as long as they are applied to the skin. Pharmaceuticals include external preparations for use against allergy or against dermatitis, especially against pollen dermatitis, and oral agents for use against allergy or against dermatitis, especially against pollen dermatitis.

The content of glutathione or a salt thereof in the composition according to the present invention or the thrombin action inhibitor can be, for example, any content included in the range of 0.0001 to 20% by weight based on the total amount of the final product such as the above-mentioned skin toner and skin serum. For example, the composition according to the present invention comprises at least 0.0001% by weight, 0.001% by weight, 0.01% by weight, 0.1% by weight, 1% by weight, or 10% by weight of glutathione or a salt thereof. Further, for example, the composition according to the present invention is less than 20% by weight, less than 1% by weight, less than 0.1% by weight, less than 0.01% by weight, less than 0.001% by weight, less than 0.001% by weight, or less than 0.0001% by weight of glutathione or a salt thereof. Depending on the properties of the final product, any combination of these upper and lower limits may be defined as a content range of glutathione or a salt thereof.

The composition or the thrombin action inhibitor according to the present invention is preferably blended as an external preparation for skin that can be directly applied to the skin. In addition to glutathione or a salt thereof, an optional compounding ingredient used in cosmetics, pharmaceuticals and the like can be appropriately blended in such an external preparation for skin, as described in the present specification. In addition, external skin preparations include external solid preparations, external powders, external liquids, liniments, sprays, external aerosols, pump sprays, ointments, creams, gels, patches, tapes, poultices, and the like; and they can be prepared in any form.

The composition or the thrombin action inhibitor according to the present invention can appropriately contain any compounding ingredients used in cosmetics, pharmaceuticals and the like as long as the effects are not impaired. Examples of the optional compounding ingredients include oils, surfactants, powders, coloring materials, water, alcohols, thickeners, chelating agents, silicones, antioxidants, ultraviolet absorbers, moisturizers, fragrances, and various medicinal ingredients, preservatives, pH regulators, neutralizers and the like. As other components, for example, an anti-inflammatory component, a whitening component, or the like may be included.

One aspect of the present invention relates to a cosmetic or non-therapeutic method for preventing skin roughness caused by pollen, which comprises applying an external preparation for skin comprising glutathione or a salt thereof.

The compositions or external skin preparations disclosed in the present specification can be used in cosmetic or non-therapeutic methods to prevent, suppress, or reduce pollen-induced skin roughness. In the cosmetic or non-therapeutic method according to the present invention, the above-mentioned external skin preparation is applied to a site where a desired effect is intended, for example, the face, neck, hands, arms, legs and the like. The application can be performed in any manner, such as application by hand or an application tool. The number of applications, frequency, amount, and the like are appropriately set according to the desired effect and the like. The cosmetic or non-therapeutic method according to the present invention does not include the so-called medical practice.

In addition, one aspect of the present invention relates to a cosmetic or non-therapeutic method for preventing skin roughness caused by pollen, which comprises applying a thrombin action inhibitor comprising glutathione or a salt thereof. Thrombin action inhibitors and the like used in such methods are described in the present specification. The form of application and the like may be determined as appropriate based on the disclosure of the present specification.

Another aspect of the present invention relates to a cosmetic or non-therapeutic method for preventing pollen-induced skin roughness, including applying a composition comprising glutathione or a salt thereof. Compositions and the like used in such methods are described herein. The form of application and the like may be determined as appropriate based on the disclosure of this specification.

Furthermore, one aspect of the present invention relates to the use of glutathione or a salt thereof to prevent skin roughness caused by pollen. From another standpoint, aspects of the present invention include glutathione or salts thereof for use in the prevention of pollen-induced skin roughness. The data disclosed in the present specification demonstrate that glutathione is useful in preventing pollen-induced skin roughness. Therefore, glutathione or a salt thereof can be used to prepare an external skin preparation or other composition for use in preventing skin roughness caused by pollen. That is, one aspect of the present invention also relates to the use of glutathione or a salt thereof in the production of an external skin preparation for preventing skin roughness caused by pollen.

The examples of the present invention described below are for illustration purposes only and do not limit the technical scope of the present invention. The technical scope of the present invention is limited only by the claims.

### Examples

### Example 1: Measurement of thrombin activity

A Rox prothrombin kit (Distributor: Rossix) was used for the measurement of thrombin activity. After glutathione (Distributor: Nacalai Tesque, Inc.) was added to a 40 ng/ml solution of prothrombin (Distributor: Invitrogen) at a concentration of 500 µg/ml to 2.5 mg/ml, the thrombin activator and thrombin substrate included in the kit were added and thrombin activity was measured. For the measurement, the substrate decomposition was observed at an absorbance of 405 nm every 40 seconds, and the substrate decomposition rates were compared.

The results are shown in Figure 1. Glutathione is found to inhibit thrombin activity in a dose-dependent manner. Although only the results of reduced glutathione are shown here, similar results were obtained in experiments using oxidized glutathione.

### Example 2: Cell excitation by Cryj1 and suppression of excitation by glutathione addition

Culture keratinocytes (Distributor: Kurabo) were cultured in the EPILIFE-KG2 medium (Distributor: Kurabo) for two days in a humidified atmosphere at 37°C. For the confluent culture, the medium was replaced with EPILIFE-KG2 + Ca1.8 mM (Distributor: Kurabo), and then after 28 to 48 hours of incubation, with EPILIFE-KG2 + Ca1 containing 5 µM of Fura2 AM (Distributor: life technology), following by one hour of culturing. Then, it was replaced with a buffer (pH 7.4) containing NaCl 150 mM, KCI 5 mM, CaCl₂ 1.8 mM, MgCl₂ 1.2 mM, HEPES 25 mM, and D-glucose 10 mM, and Cryj1 (Distributor: HAYASHIBARA) was added to the buffer at the concentration of 100 ng/ml alone, or with glutathione (Distributor: Nacalai Tesque, Inc.) (5 µg/ml). Fluorescence was measured with a fluorescence microscope to measure changes in the intracellular calcium concentration. The results are shown in Figure 2.

The value of intracellular calcium concentration about 2 minutes after the addition of Cryj1 was calculated and quantified using the Aqua Cosmos software. The results are shown in Figure 3. These results demonstrate that glutathione significantly suppresses the calcium response elicited by Cryj1.

## Claims

1. Glutathione or salt thereof for use in treating or preventing skin roughness caused by pollen dermatitis.

2. Glutathione or salt thereof for use of claim 1, wherein glutathione or a salt thereof is applied to skin as an external skin preparation comprising glutathione or a salt thereof at 0.0001 to 20% by weight.

3. Glutathione or salt thereof for use of any one of claims 1 to 2, wherein the pollen is cedar pollen.

4. A non-therapeutic method for preventing skin roughness caused by pollen, comprising applying an external skin preparation comprising glutathione or a salt thereof.

5. A non-therapeutic method for preventing pollen-induced skin roughness, comprising applying a thrombin action inhibitor that comprises glutathione or a salt thereof.

## Patentansprüche

1. Glutathion oder dessen Salz zur Verwendung bei der Behandlung oder Vorbeugung von durch Pollendermatitis verursachte Hautrauheit.

2. Glutathion oder dessen Salz zur Verwendung nach Anspruch 1, wobei Glutathion oder dessen Salz als äußerliches Hautpräparat, enthaltend Glutathion oder dessen Salz in einer Konzentration von 0,0001 bis 20 Gewichts-%, auf die Haut aufgetragen wird.

3. Glutathion oder dessen Salz zur Verwendung nach einem der Ansprüche 1 bis 2, wobei es sich bei dem Pollen um Zedernpollen handelt.

4. Nicht-therapeutisches Verfahren zur Vorbeugung von durch Pollen verursachte Hautrauheit, umfassend das Auftragen eines äußerlichen Hautpräparats, enthaltend Glutathion oder dessen Salz.

5. Nicht-therapeutisches Verfahren zur Vorbeugung von polleninduzierter Hautrauheit, umfassend das Auftragen eines Thrombin-Wirkungshemmers, der Glutathion oder dessen Salz enthält.

## Revendications

1. Glutathion ou sel de celui-ci pour le traitement ou la prévention de rugosités cutanées causées par la dermatite pollinique.

2. Glutathion ou sel de celui-ci pour une utilisation selon la revendication 1, dans lequel le glutathion ou un sel de celui-ci est appliqué sur la peau en tant que préparation cutanée externe comportant du glutathion ou un sel de celui-ci à 0,0001 à 20 % en poids.

3. Glutathion ou sel de celui-ci pour une utilisation selon l'une quelconque des revendications 1 à 2, dans lequel le pollen est du pollen de cèdre.

4. Procédé non thérapeutique de prévention de rugosités cutanées causées par le pollen, comportant l'application d'une préparation cutanée externe comportant du glutathion ou un sel de celui-ci.

5. Procédé non thérapeutique de prévention de rugosités cutanées induites par le pollen, comportant l'application d'un inhibiteur d'action de la thrombine qui comporte du glutathion ou un sel de celui-ci.
